**Europäisches Patentamt**

**European Patent Office**

**Office européen des brevets**

(19)

(11) Veröffentlichungsnummer: **0 230 588**
**B1**

(12) # EUROPÄISCHE PATENTSCHRIFT

(45) Veröffentlichungstag der Patentschrift:
23.11.88

(21) Anmeldenummer: **86117207.0**

(22) Anmeldetag: **10.12.86**

(51) Int. Cl.⁴: **A 61 M 16/01**

(54) **Narkosemittelbehälter.**

(30) Priorität: **14.12.85 DE 3544302**

(43) Veröffentlichungstag der Anmeldung:
**05.08.87 Patentblatt 87/32**

(45) Bekanntmachung des Hinweises auf die Patenterteilung:
**23.11.88 Patentblatt 88/47**

(84) Benannte Vertragsstaaten:
**DE FR GB IT SE**

(56) Entgegenhaltungen:
**EP-A-0 049 067**
**DE-B-1 098 169**
**DE-B-1 566 592**
**DE-B-1 900 271**
**DE-C-712 912**
**GB-A-758 657**

(73) Patentinhaber: **Drägerwerk Aktiengesellschaft,
Moislinger Allee 53- 55, D-2400 Lübeck 1 (DE)**

(72) Erfinder: **Altner, Ulrich, Dr., Dipl.- Ing., Karl-
Storch- Strasse 11, D-2360 Bad Segeberg (DE)**
Erfinder: **Falb, Wolfgang, Dipl.- Ing., Harmskamp
5, D-2061 Klein Wesenberg (DE)**
Erfinder: **Ryschka, Martin, Dr., Dipl.- Ing.,
Morierstrasse 10b, D-2406 Stockelsdorf (DE)**
Erfinder: **Wallroth, Carl- Friedrich, Dr., Dipl.- Ing.,
Stresemannstrasse 1, D-2400 Lübeck (DE)**

## Beschreibung

Die Erfindung betrifft einen Behälter für flüssige Narkosemittel, dessen Seitenwand zumindest eine Zulauföffnung zur Befüllung und eine Überlauföffnung aufweist.

Flüssige Narkosemittel werden in der Medizin häufig durch einen Narkosemittelverdunster in die Atemgasleitung des zu narkotisierenden Patienten geführt. Ein Vorratsbehälter mit Narkosemittel an dem Verdunster ermöglicht einen längeren Einsatz des Verdunsters während einer Narkose. Das Befüllen des Narkosemittelbehälters erfolgt vor einem Einsatz oder nach einer mehr oder weniger längeren Betriebszeit des Narkosemittelverdunsters. Um ein Überfüllen des Narkosemittelbehälters zu vermeiden, wird eine Überlaufsicherung an dem Narkosemittelbehälter angebracht. Eine derartige Überlaufsicherung ist in der DE-Betriebsanleitung 5327.09, Feb. 83: "Sicherheitsfülleinrichtung zum Vapor 19.1" der Firma Drägerwerk AG, Lübeck, DE, angegeben. An dem Behälter des Narkosemittelverdunsters ist seitlich eine Fülleinrichtung angeordnet, an welcher über einen Anschlußstutzen der Befüllschlauch einer Narkosemittelflasche angeschlossen werden kann. Der Befüllschlauch besitzt ein Kupplungsstück, welches an den Anschlußstutzen angekoppelt werden kann. In dem Kupplungsstück und dem Befüllschlauch befindet sich der Füllkanal für das Narkosemittel und der Entlüftungskanal zur Belüftung der Narkosemittelflasche während des Einfüllens des Narkosemittels in den Narkosemittelbehälter. Bei eingekoppeltem Füllschlauch setzen sich der Füllkanal und der Entlüftungskanal in der Fülleinrichtung fort und münden als Zulauf und als Entlüftungsöffnung seitlich in den Narkosemittelbehälter. Beim Einfüllen des Narkosemittels aus der Narkosemittelflasche in den Narkosemittelbehälter tritt die Flüssigkeit aus dem Zulauf aus, und die in dem Narkosemittelbehälter vorhandene Luft wird über die Entlüftungsöffnung in die Narkosemittelflasche zurückgeleitet. Bei Erreichen eines vorgegebenen maximalen Füllstandes wird die Entlüftungsöffnung verschlossen, und die Befüllung aus der Narkosemittelflasche ist beendet. Sollte jedoch aufgrund eines Fehlerfalles, wie z. B. einer fehlerhaften Dichtung im Füllschlauch, eine weitere über den maximalen Füllstand hinausgehende Befüllung des Narkosemittelbehälters erfolgen, ist ein weiterer Überlauf an dem Behälter angeordnet, durch welchen das überschüssige Narkosemittel austreten kann.

Wird nun ein gefüllter Narkosemittelbehälter in seiner Lage derart geneigt, daß der Flüssigkeitsspiegel den Zulauf und den Überlauf bedeckt, oder schwappt infolge einer Stoßbewegung die Flüssigkeit in den Zulauf und den Überlauf, so kann eine Restmenge an Narkosemittelflüssigkeit aus Teilabschnitten der Kanalführung in der Fülleinrichtung nicht beseitigt werden. Sie fließt entweder sogleich oder nach Öffnen eines Verschlusses heraus und belästigt die sich in der Umgebung aufhaltenden Personen.

Der vorliegenden Erfindung liegt somit die Aufgabe zugrunde, eine Überlaufsicherung der genannten Art so zu verbessern, daß auch bei Verlagerungen oder Schwingungen des Flüssigkeitsspiegels im Narkosemittelbehälter ein Eindringen von Narkosemittelflüssigkeit in den Zulauf und den Überlauf verhindert wird.

Zur Lösung dieser Aufgabe ist vorgesehen, daß sich von der Zulauföffnung und der Überlauföffnung jeweils ein zur Bodenfläche des Behälters hin geneigtes Rohrstück erstreckt, deren Mündungen in der Nähe der der Zulauf- und Überlauföffnungen gegenüberliegenden Wand des Behälters liegen.

Die mit der Erfindung erzielten Vorteile liegen insbesondere darin, daß bei geringen Neigungen um die vertikale Achse des Narkosemittelbehälters, insbesondere jedoch bei durch Stöße verursachten Verlagerungen des Flüssigkeitsspiegels die Flüssigkeit allenfalls in das freie Rohrende der Rohrstücke bis zu einer gewissen Länge eindringen kann, jedoch durch deren Neigung gezwungen wird, in den Narkosemittelbehälter zurückzufließen. Eine Überflutung der Kanalführung in der Fülleinrichtung wird dadurch vermieden. Die Rohrstücke können gestreckt verlaufen, es ist jedoch auch möglich, gebogene, wendelförmige oder ähnliche Umwegführungen zu verwenden.

Durch die Abwinklung der Mündungen der Rohrstücke wird ein direktes Eindringen von Flüssigkeitsspritzern bis tief in die Rohrstücke verhindert.

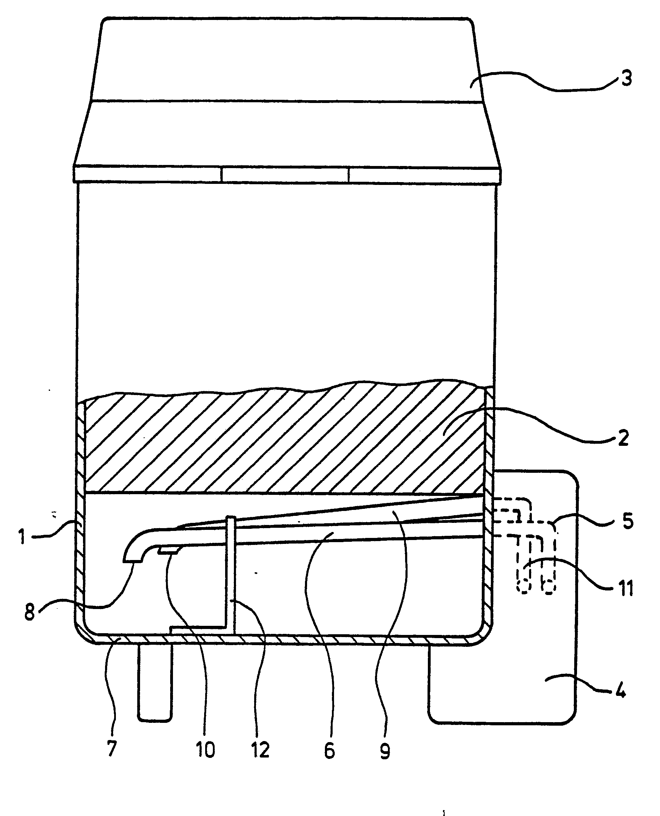

Ein Ausführungsbeispiel der Erfindung wird anhand der Zeichnung dargestellt und im folgenden näher erläutert.

Die einzige Figur zeigt schematisch einen teilgeschnittenen Narkosemittelverdunster, der in seinem unteren Teil den Narkosemittelbehälter 1 und in seinem oberen Teil den schraffiert dargestellten Verdunsterblock 2 enthält. Mit einem Handrad 3 kann die abgegebene Narkosemittelkonzentration in bekannter Weise eingestellt werden. An die Außenwand des Narkosemittelbehälters 1 ist eine Fülleinrichtung 4 befestigt, durch deren Zulauf 5 das Narkosemittel über das Rohrstück 6 und dessen zur Bodenfläche 7 hin geneigte Mündung 8 in den Innenraum des Narkosemittelbehälters 1 gefüllt wird. Das Rohrstück 9 mit seiner ebenfalls zur Bodenfläche 7 hin geneigten Mündung 10 ist mit dem Überlauf 11 der Fülleinrichtung 4 verbunden. Die Mündungen 8, 10 liegen in der Nähe der dem Zulauf 5 und Überlauf 11 gegenüberliegenden Wand des Behälters 1. Beide Rohrstücke 6, 9 sind über eine Halterung 12 an der Bodenfläche 7 befestigt.

**Patentansprüche**

1. Behälter (1) für flüssige Narkosemittel, dessen Seitenwand zumindest eine Zulauföffnung (5) zur Befüllung und eine Überlauföffnung (11) aufweist, dadurch gekennzeichnet, daß sich von der Zulauföffnung (5) und der Überlauföffnung (11) jeweils ein zur Bodenfläche (7) des Behälters (1) hin geneigtes Rohrstück (6, 9) erstrecken, deren Mündungen (8, 10) in der Nähe der der Zulauf- (5) und Überlauföffnungen (11) gegenüberliegenden Wand des Behälters (1) liegen.

2. Behälter nach Anspruch 1, <u>dadurch gekennzeichnet</u>, daß die Mündungen (8, 10) zur Bodenfläche (7) hin abgewinkelt sind.

**Claims**

1. Container (1) for fluid anaesthetic, the side wall of which container has at least one inflow opening (5) for filling purposes and an overflow opening (11), characterised in that there extends from the inflow opening (5) and the overflow opening (11), in each case, a pipe piece (6, 9) which is inclined towards the bottom surface (7) of the container (1) and the mouths (8, 10) of which lie near the wall of the container (1) lying opposite the inflow (5) and overflow openings (11).

2. Container according to claim 1, characterised in that the mouths (8, 10) are bent at an angle towards the bottom surface (7).

**Revendications**

1. Récipient (1) pour agents narcotiques, dont la paroi latérale présente au moins un orifice d'admission (5) pour le remplissage et un orifice de trop-plein (11), caractérisé en ce que des éléments tubulaires respectifs (6, 9) inclinés vers la face de fond (7) du récipient (1) s'étendent à partir de l'orifice d'admission (5) et de l'orifice de trop-plein (11), éléments dont les embouchures (8, 10) se situent à proximité de la paroi du récipient (1) qui est opposée aux orifices d'admission (5) et de trop-plein (11).

2. Récipient selon la revendication 1, caractérisé en ce que les embouchures (8, 10) sont coudées vers la face de fond (7).